# EUROPÄISCHE PATENTANMELDUNG

(11) **EP 2 422 777 A1**
(43) Veröffentlichungstag der Anmeldung: **29.02.2012**
(21) Anmeldenummer: 10173500.9
(22) Anmeldetag: 20.08.2010
(51) Int. Cl.: A61K 31/05, A61K 31/198, A61K 31/53

(54) **Synergistische Wirkung von Modulatoren des NO-Stoffwechsels und der NADPH-Oxidase bei der Sensitivierung von Tumorzellen**

(71) Anmelder: Universitätsklinikum Freiburg, 79106 Freiburg (DE)
(72) Erfinder: Bauer, Georg, 79104, Freiburg (DE)
(74) Vertreter: Keller, Günter

(57) **Zusammenfassung**

Offenbart werden pharmazeutische Zusammensetzungen, die eine pharmazeutisch wirksame Menge an wenigstens einem Wirkstoff enthalten, der die verfügbare NO-Konzentration in der Zelle erhöht, zusammen mit wenigstens einem Wirkstoff, der die NADPH-Oxidase stimuliert.

## Beschreibung

Gegenstand der vorliegenden Anmeldung sind Mittel, die zur Bekämpfung von Tumoren eingesetzt werden können. Es handelt sich hierbei um Wirkstoffe, die spezifisch in den Stoffwechsel der Tumorzellen eingreifen und zu einer Apoptose der Tumorzellen führen.

Die vorliegende Erfindung geht davon aus, dass die autokrine, durch Reaktive Sauerstoffspezies (ROS) vermittelte Apoptoseinduktion in transformierten Rattenfibroblasten (208Fsrc3), als Modell für maligne Zellen *zunächst durch kleine* Konzentrationen von Cu- oder Mn-haltiger Superoxiddismutase (Cu-SOD oder Mn-SOD) gehemmt wird. Dies beruht auf der notwendigen Beteiligung von Superoxidanionen an der durch den HOCI-Weg bedingten Apoptoseinduktion. Zentrale Inhibitoren wie Taurin (HOCI), ABH (Peroxidase) und Mannitol (Hydroxylradikale) belegen die Wirkung des HOCI-Wegs. Nach Erreichen der maximalen Hemmwirkung durch beide Formen der SOD erfolgt dann aber spezifisch für die Cu-SOD eine konzentrationsabhängige Apoptoseinduktion im höheren Konzentrationsbereich. Diese beruht auf den besonderen elektrochemischen Fähigkeiten des Cu-lons im Enzym. Im ersten Reaktionsschritt reagiert Cu⁺⁺-SOD mit dem einem Superoxidanion unter Bildung von Sauerstoff und des Enzymintermediates mit einem einwertigen Kupferion:

Cu⁺⁺-SOD + O₂⁻ → Cu⁺-SOD + O₂ 1)

Im zweiten Reaktionsschritt bildet das Enzymintermediat aus einem zweiten Superoxidanion und zwei Protonen Wasserstoffperoxid und die Ausgangsform des Enzyms mit zweiwertigem Kupfer wird wieder hergestellt:

Cu⁺-SOD + O₂⁻+ 2 H+ → Cu⁺⁺-SOD + H₂O₂ 2)

Bei einer hohen CuSOD-Konzentration findet nicht jedes Cu⁺-SOD-Intermediat ein Superoxidanion. Alternativ führt es dafür mit HOCI eine Fenton-ähnliche Reaktion durch, bei der ein Elektron vom Intermediat auf HOCI übertragen wird und dadurch die Cu⁺⁺-Form des Enzyms wiederhergestellt ist, aber aus HOCI Chloridionen und Apoptose-auslösende Hydroxylradikale entstehen:

Cu⁺-SOD + HOCl → Cu⁺⁺-SOD + Cl⁻ + OH 3)

Reaktion 3) ist also für den Wiederanstieg der Aptoseinduktion bei höheren Cu-SOD-Konzentrationen verantwortlich, wie die Inhibitordaten zeigen. Mn-SOD kann diese Reaktion nicht durchführen, da das Manganion im Gegensatz zu Kupfer- und Eisenionen nicht zur Fentonreaktion befähigt ist.

Die Ausbildung einer Glockenkurve bei der Hemmung durch Cu-SOD ist nicht nur radikalchemisch interessant, sondern bietet auch die Grundlage für die Quantifizierung von Superoxidanionen. Denn aus den Grundlagen der Reaktion lässt sich ableiten, dass jede Veränderung der Superoxidanionenkonzentration im System zu einer gut detektierbaren Verschiebung der Glockenkurve führen sollte, wobei der Scheitelpunkt ein gutes Werkzeug für eine präzise Messung darstellt.

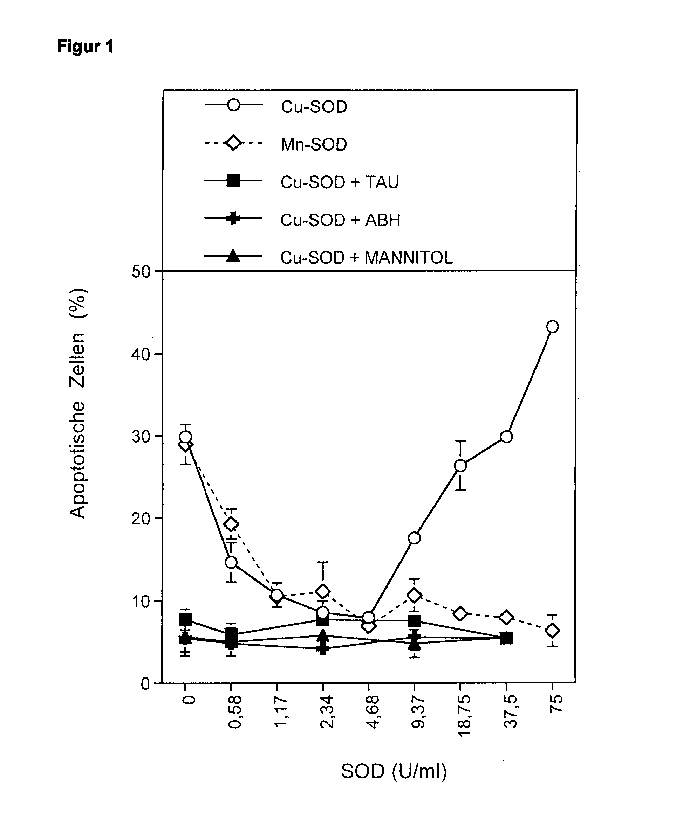

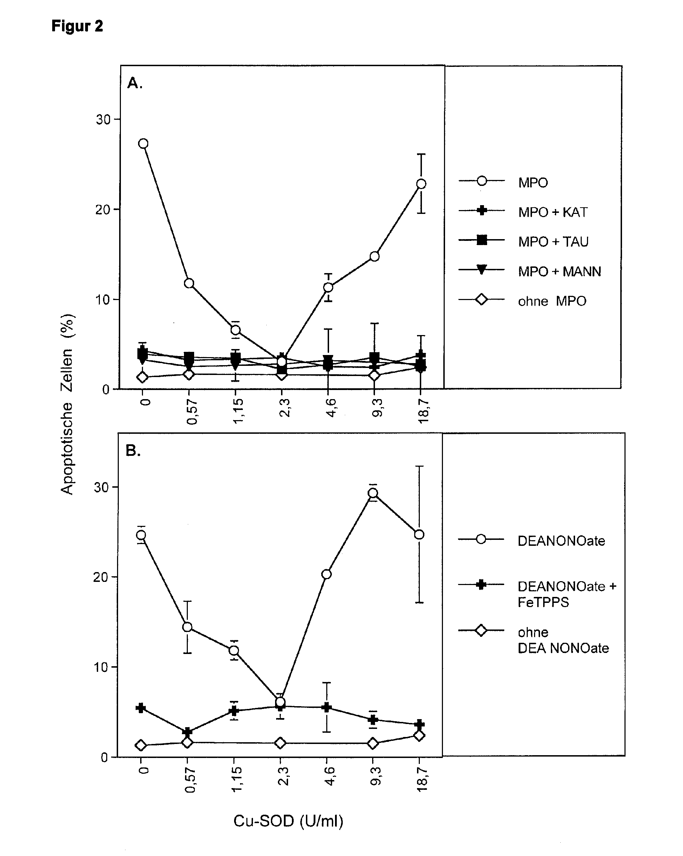

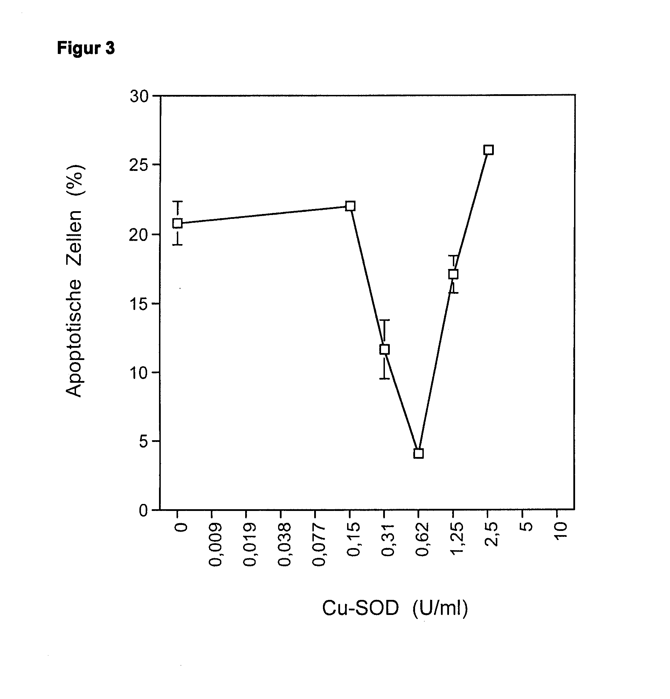

In den Figuren 2A, 2B und 3 werden weitere Belege für diese charakteristische Reaktion der Cu-SOD vorgestellt. In Figur 2A wird der HOCl-Weg bei 208Fsrc-Zellen durch die Zugabe exogener Myeloperoxidase (MPO) forciert und dadurch im Vergleich zur in Figur 1 gezeigten autokrinen Apoptoseinduktion ohne exogene Zusätze wesentlich beschleunigt (ein experimentell-technischer Vorteil). Die chemischen Abläufe in den in Figur 1 und 2A vorgestellten Experimenten sind identisch.

In dem in Beispiel 2, Figur 2B vorgestellten Experiment wird in 208Fsrc3-Zellen durch Zugabe eines NO-Donors (DEANONOATE) der NO/Peroxynitritweg induziert. Auch hier ergibt sich die charakteristische Glockenkurve für Cu-SOD. Die zugrunde liegende Chemie ist aber verschieden von den vorher genannten Beispielen. Auf der linken Seite der Kurve verhindert CuSOD die Bildung von Peroxynitrit, indem sie die für die Reaktion mit NO benötigten Superoxidanionen wegfängt. Im rechten Teil der Glockenkurve führt das Intermediat mit dem einwertigen Kupfer, da es kein zweites Superoxidanion erreicht, zur Reduktion von NO zum Nitroxylanion. Dieses reagiert mit Luftsauerstoff zu Peroxynitrit, das Apoptose auslöst:

Cu⁺⁺-SOD + O₂⁻ → Cu⁺-SOD + O₂ 1)

Cu⁺-SOD + NO → Cu⁺⁺-SOD + NO- 2)

NO- + O₂ → ONOO⁻ 3)

Das in Figur 3 gezeigte Beispiel der Cu-SOD-Glockenkurve beruht auf der bisher unveröffentlichten HOCl-Synthese hoher Konzentrationen des Salen-Mangan-Komplexes EUK-8 (Manganese N,N'-bis(salicylidiene)-ethylenediamine chloride), der eigentlich als Katalasemimetikum bekannt ist.

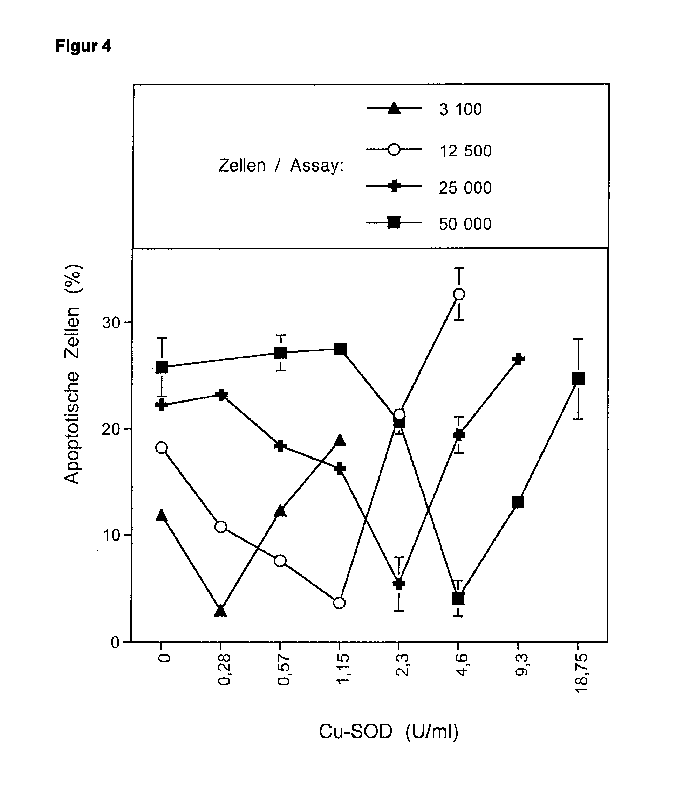

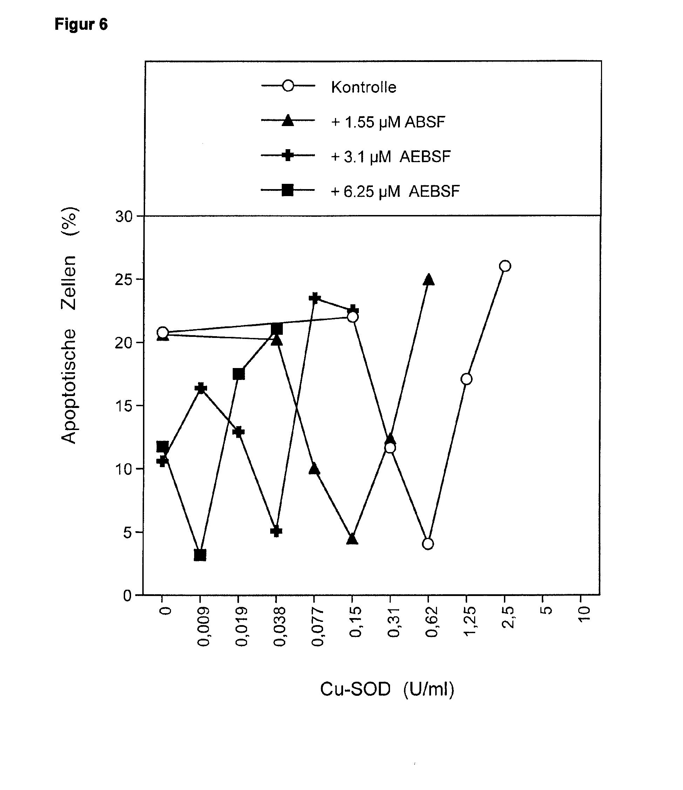

Der Stoff wirkt also in hohen Konzentrationen wie eine HOCI-synthetisierende Peroxidase. Der Vorteil dieses Systems liegt darin begründet, dass dieser Stoff eine höhere Affinität für das Substrat Wasserstoffperoxid zeigt als natürliche Peroxidasen. Die Reaktion erfolgt daher auch bei sehr geringen Wasserstoffperoxidkonzentrationen. Die Messungen, die in Figuren 4 oder 6 gezeigt werden, verdanken ihre Machbarkeit diesem spezifischem Induktor der Apoptose.

Es stehen also verschiedene Apoptoseinduktionssyteme zur Verfügung, die unterschiedlichen experimentellen Anforderungen zur Bestimmung der Superoxidanionenbeteiligung und deren relativer Konzentration gerecht werden können. Dabei belegt die gleichsinnige Wirkung der CuSOD (nicht aber der MnSOD) in den verschiedenen Systemen, dass die Erklärung für die chemischen Abläufe, die auf der einwertigen Cu-SOD beruhen, zutreffend sein sollten.

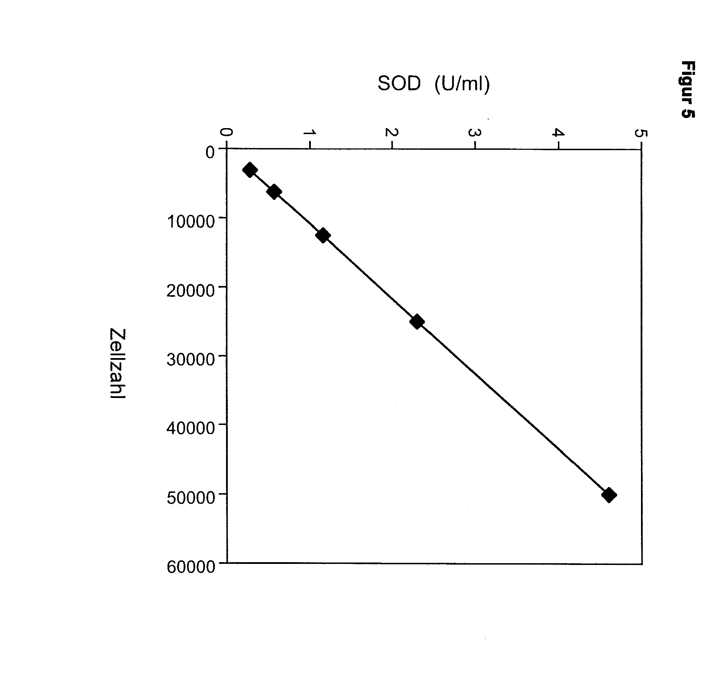

Mit der Figur 4 kann eine Kalibrationskurve für die Messung der relativen Superoxidanionenkonzentration durch SOD erstellt werden. Im Ansatz befanden sich unterschiedliche Mengen an Superoxidanionen-produzierenden Zellen, damit also variierende Konzentrationen an Superoxidanionen. Die Glockenkurven der Hemmung durch CuSOD wurden wie erwartet verschoben. Die in Figur 5 gezeigte Eichkurve, ermittelt aus Figur 4, zeigt strenge Linearität.

Eine zweite Bestätigung der Leistungsfähigkeit dieses Mess-Systems wird in Figur 6 gezeigt. Hier führt die partielle Hemmung der NADPH-Oxidase durch AEBSF (4-(2-Aminoethyl)-benzenesulfonyl fluoride) zu klaren Verschiebungen der Kurven, was aufgrund der Hemmung der NADPH-Oxidase auch erwartet werden konnte.

Die Anwendung der Messung der Superoxidanionenkonzentration durch CuSOD wird in den folgenden Beispiele bestätigt und dazu verwendet, zu prüfen, ob bestimmte Einwirkungen oder Stoffe die Superoxidanionenproduktion durch NADPH-Oxidase beeinflussen.

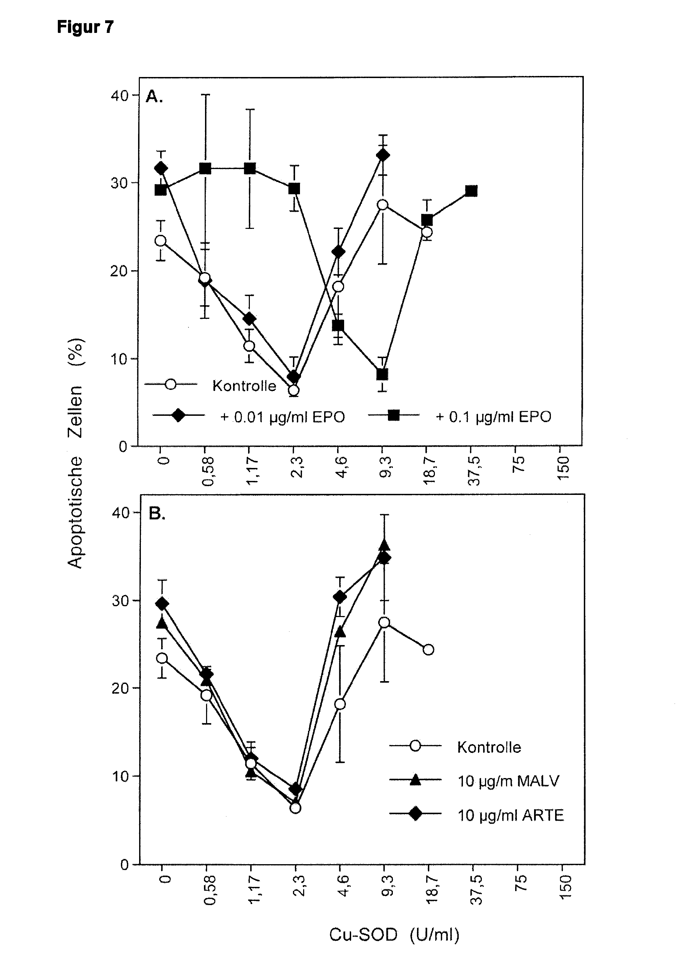

Figur 7 zeigt, dass bei der für das Wirkoptimum charakteristischen Konzentration von Epothilon B eine gewisse Stimulierung der NADPH-Oxidase nachgewiesen werden kann (vierfach), dies bei niedrigeren Konzentrationen aber nicht mehr der Fall ist. (Ähnlich verhält sich auch Taxol, nicht gezeigt). Substanzen wie Malvidinchlorid oder Artemisinin zeigen jedoch keinerlei Wirkung auf die NADPH-Oxidase.

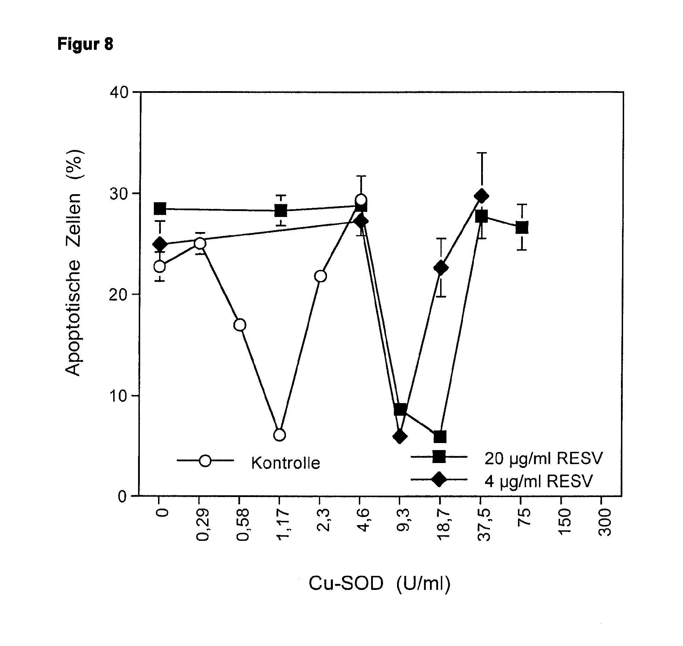

Resveratrol zeigt eine sehr starke Stimulierung der NADPH-Oxidase (Abbildung 8).

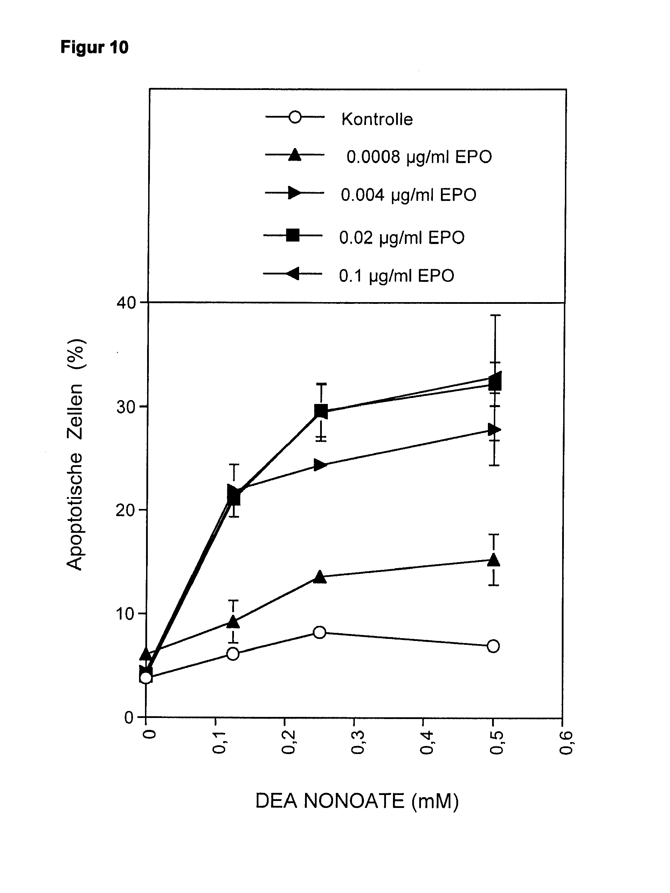

Schließlich belegt Figur 10, dass die Aktivierung des FAS-Rezeptors durch monoklonale Antikörper gegen den Rezeptor (A) oder durch Singulettsauerstoff (B), erzeugt durch die Belichtung des Photosensitizers Photofrin, zu einer deutlichen Stimulierung der NADPH-Oxidase führt. Diese Aussage wird dadurch bestärkt, dass die Hemmung der dem FAS-Rezeptor direkt nachgeschalteten Caspase-8 zu einer Hemmung der Stimulierung der NADPH-Oxidase führt.

### Hemmung der NO-Dioxygenase (NOD)

Dieser Test beruht darauf, dass NOD auch exogen zugegebenes NO effektiv in Nitrat umwandelt und damit in geeigneten Zellsystemen (wie z. B. der Tumorzelllinie MKN-45) die Apoptoseinduktion durch NO/Peroxynitrit unterbinden kann.

Voraussetzung ist eine dichte Zellkultur der Tumorzellen, deren Katalase durch die Gabe von 200 mM 3-AT vollständig gehemmt wird. Dies schließt aus, dass eine Testsubstanz über die Modulation der Katalaseaktivität auf die Gesamtreaktion Einfluss nehmen könnte. Das nach Katalasehemmung frei gesetzte Wasserstoffperoxid wird durch 20 - 25 µM des Katalasemimetikums EUK-134 (analog zu dem vorher erwähnten EUK-8, jedoch mit geringeren Peroxidaseaktivität) vollständig abgebaut. Dadurch wird sowohl der HOCl-Weg als auch der Konsum von NO durch Wasserstoffperoxid verhindert. Nun bleibt für die Modulation der verfügbaren NO-Konzentration unter den bekannten Mechanismen nur noch die NOD. Wird sie gehemmt, so führt die Zugabe von exogenem NO zu einer gesteigerten Apoptoseinduktion.

Figur 10 zeigt, dass Epothilon B (EPO) über einen sehr weiten Konzentrationsbereich sehr effektiv zu einer Erhöhung der verfügbaren NO-Konzentration führt, die am besten durch NOD-Hemmung erklärt werden kann.

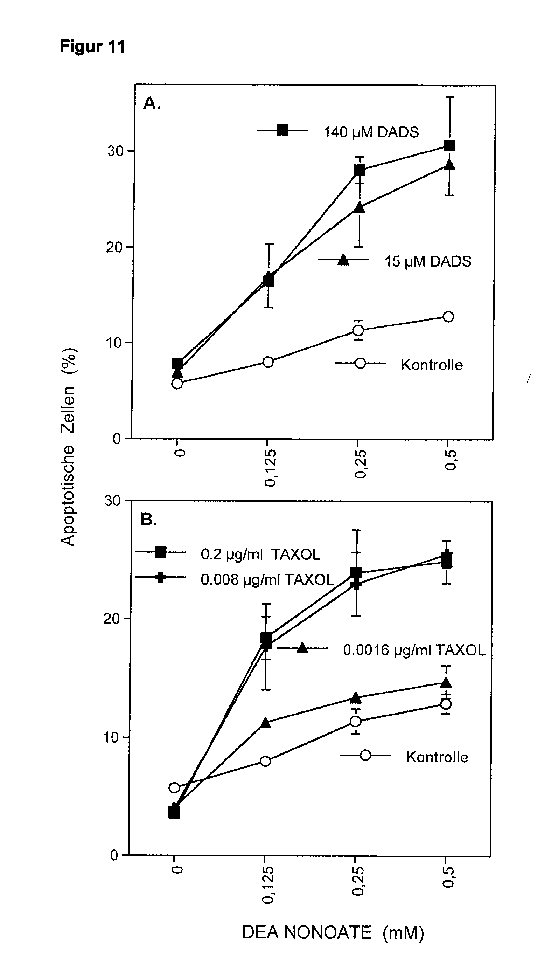

Der gleiche Befund ergibt sich für Diallydisulfid (DADS) und Taxol (Abbildung 11). Für Resveratrol ergaben sich keine Anhaltspunkte für eine Hemmfähigkeit der NOD (Daten nicht gezeigt).

Gegenstand der vorliegenden Erfindung sind daher pharmazeutische Zusammensetzungen, die eine pharmazeutisch wirksame Menge an wenigstens einem Wirkstoff enthalten, der die verfügbare NO-Konzentration in der Zelle erhöht, zusammen mit einem Wirkstoff, der die NADPH-Oxidase stimuliert.

In bevorzugter Ausführungsform ist der Wirkstoff, der die NADPH-Oxidase stimuliert, ausgewählt aus Resveratrol, Transforming growth factor-beta (TGF-ß) und/oder Angiotensin II. TGF-ß gehört zu den Signalmolekülen. Die TGF-ß Polypeptide sind multifunktional und können die Zellproliferation beeinflussen. Angiotensin II ist ein Oktapeptid und zählt zu den Gewebshormonen.

In einer besonders bevorzugten Ausführungsform beinhaltet die pharmazeutische Zusammensetzung als Wirkstoff, der die NADPH-Oxidase stimuliert, die Verbindung Resveratrol. Resveratrol ist ein zu den Polyphenolen gehörender Wirkstoff mit antioxidativen Eigenschaften. Chemisch gesehen ist Resveratrol ein Stilbenoid. Resveratrol kommt als trans- oder cis-Isomer vor. Erfindungsgemäß werden beide Isomere eingesetzt. Resveratrol findet sich in verschiedenen Pflanzen, bzw. Lebensmitteln die aus diesen Pflanzen gewonnen wurden. Insbesondere sind hier Weintrauben, Himbeeren, Pflaumen und Erdnüsse zu erwähnen.

Erfindungsgemäß bevorzugt ist, dass der Wirkstoff, der die verfügbare NO-Konzentration in der Zelle erhöht, keine gleichzeitige Wirkung auf die NADPH-Oxidase ausübt.

Der andere Wirkstoff in den erfindungsgemäßen pharmazeutischen Zusammensetzungen ist ein Wirkstoff, der die NO-Konzentration in der Zelle steigert. Ein derartiger Wirkstoff kann ausgewählt sein aus Arginin und/oder Arginasehemmstoffen, insbesondere NOHA und oder NOR-NOHA.

In einer weiteren bevorzugten Ausführungsform ist der Wirkstoff, der die verfügbare NO-Konzentration in der Zelle erhöht, ein Stoff, der die NO-Synthase induziert.

Ein bevorzugter Wirkstoff, der die NO-Synthase induziert, ist Interferon Y.

In weiterer bevorzugter Ausführungsform ist der Wirkstoff, der die verfügbare NO-Konzentration in der Zelle erhöht, ein Stoff, der eine hemmende Wirkung auf die NO-Dioxigenase hat und ausgewählt ist aus
a) Flavonoiden, insbesondere
   Xanthohumol, Isoxanthohumol, 6-Prenylnaringenin,
   8-Prenylnaringenin, Quercetin, Quercitrin, Isoquercetin, Rutin,
   Taxifolin, Hyperosid, und/oder
b) Anthocyanen, insbesondere
   Cyandidinchlorid, Malvidinchlorid, Malvidin-3-O-galaktosid,
   Pelargonin, Peonidinchlorid, Pelargonidin, und/oder
c) Fettsäuren, insbesondere
   Palmitinsäure, Stearinsäure, Myristinsäure, und/oder
d) Azolen, insbesondere
   Biconazol, Econazol, Fluconazol, Itraconazol, Ketoconazol,
   Miconazol; Sulconazol, und/oder
e) Artemisinin, Chloroquin, Primaquin.

Die erfindungsgemäßen Zusammensetzungen werden bevorzugt verwendet zur Behandlung von Magenkrebs, Prostatakrebs und/oder Brustkrebs.

Die Beispiele und Figuren zeigen synergistische Wirkungen zwischen Wirkstoffen, die die verfügbare NO-Konzentration erhöhen und solchen, die die NADPH-Oxidase stimulieren.

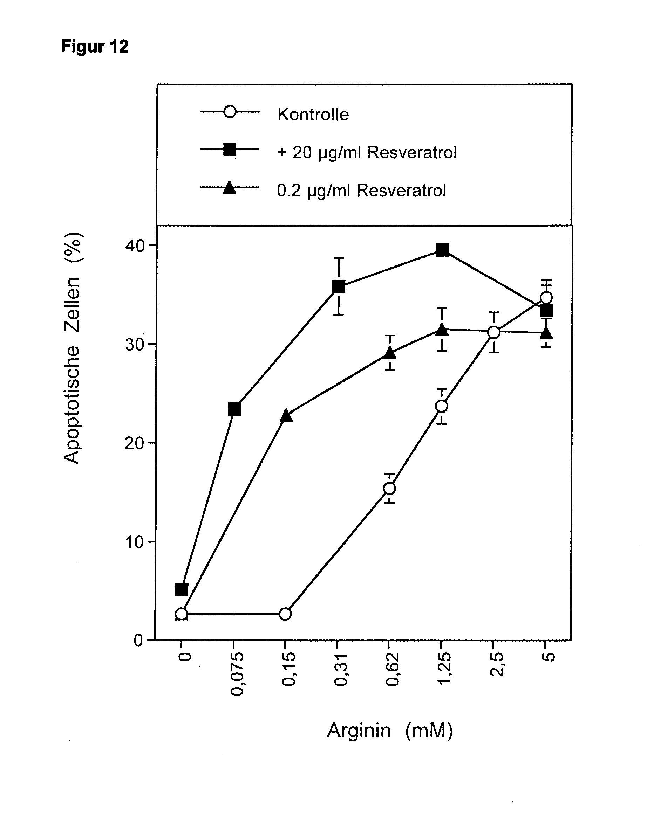

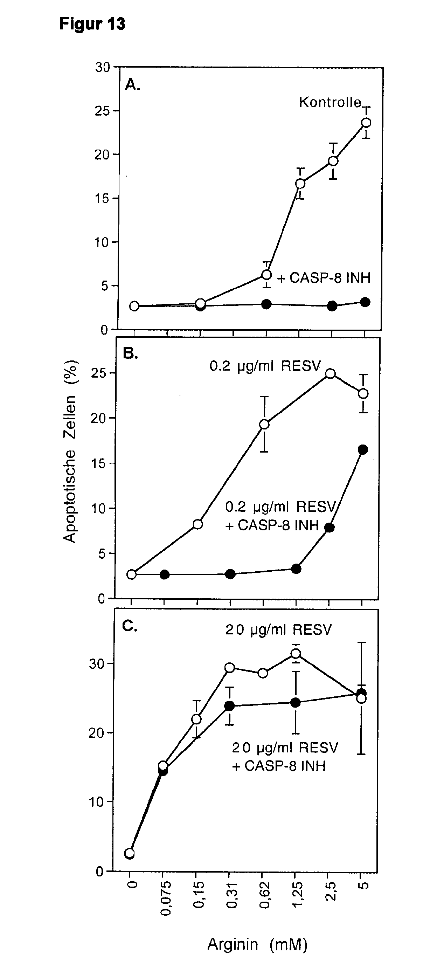

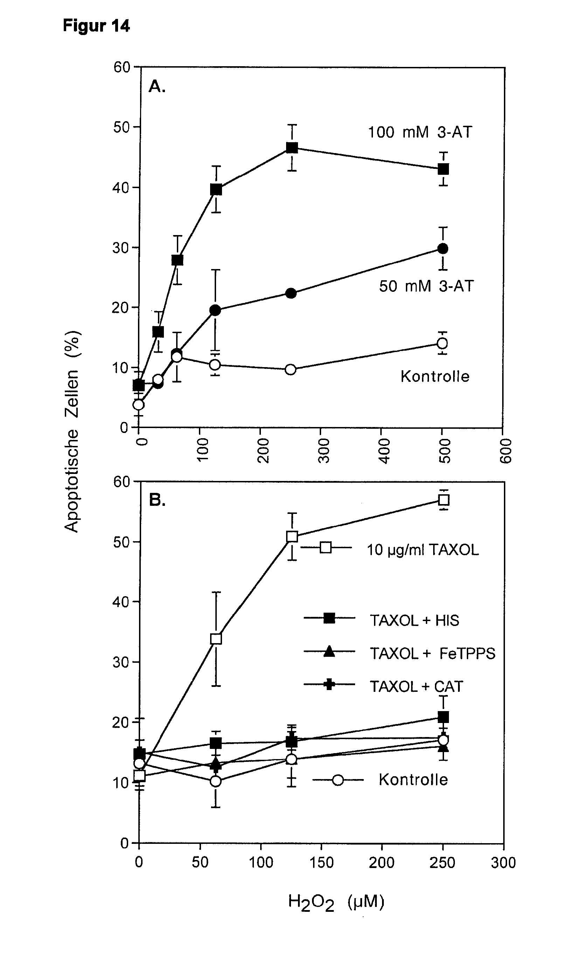

Die Figuren 12 und 13 belegen, dass eine Erhöhung der Argininkonzentration (Substrat der NO-Synthase) Apoptose induziert. Daten, die belegen, dass dies auf der Erhöhung des NO-Spiegels, vermehrter Peroxynitritbildung, Singulettsauerstoffbildung und Inaktivierung der Katalase beruht, sind hier nicht gezeigt. In niedrigen Konzentrationen von Arginin wird ein sehr beeindruckender synergistischer Effekt mit Resveratrol erkennbar (Figur 12). Figuren 13 und 14 zeigen, dass die Wirkung von Arginin alleine von der Amplifikation durch das FAS-System (Steigerung der Superoxidanionproduktion) abhängig ist, da Caspase-8-Inhibitor die Arginin-vermittelte Apoptoseinduktion vollständig blockieren kann. Resveratrol kann diesen FAS-abhängigen Amplifikationsschritt ersetzen.

Die Singulettsauerstoff-vermittelte Inaktivierung der Tumorzellkatalase nach Einwirken von Taxol wird in Beispiel 14 direkt demonstriert. Dafür sind Peroxynitrit (hemmbar durch FeTPPS) und Wasserstoffperoxid (hemmbar durch Katalase) und deren bekanntes Reaktionsprodukt Singulettsauerstoff (gehemmt durch Histidin) verantwortlich.

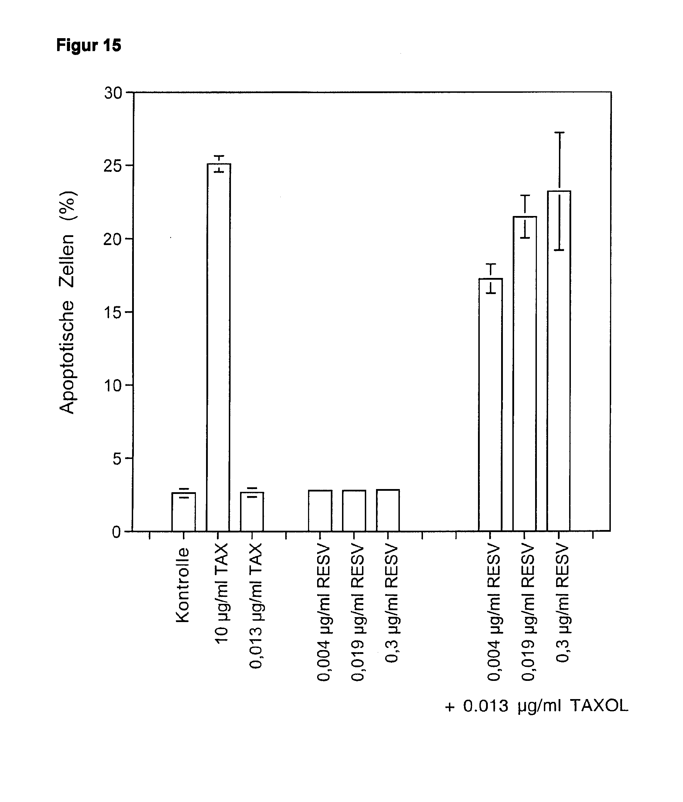

Ein synergistischer Effekt mit Taxol ergibt sich auch, mit dem NADPH-Oxidasestimulator Resveratrol (Figur 15).

Weitere Beispiele für synergistische Effekte sind:

Figur 16: Epothilon B (NOD-Hemmer) und Resveratrol (NADPH-Oxidase-Stimulator);

Figur 17: Cyanidinchlorid (NOD-Hemmer) und Resveratrol (NADPH-Oxidase-Stimulator).

Beobachtet wurde eine Steigerung der verfügbaren NO-Konzentration durch Hemmung der NOD in einem weiten Konzentrationsbereich und gleichzeitige Stimulierung der NADPH-Oxidase im oberen Konzentrationsbereich der Stoffe:

Taxol, Epothilon B, Allylisothiocyanat

Diese Stoffgruppe zeichnet sich dadurch aus, dass im Bereich der optimalen Wirkkonzentration keine zusätzliche Stimulation durch weitere Substanzen notwendig ist. Die Sensitivierung der Tumorzellen im optimalen Konzentrationsbereich dieser Stoffgruppe läuft ohne Amplifikationsschritte durch das FAS-Rezeptorsystem. Im niedrigeren Konzentrationsbereich wird das FAS-System dazu geschaltet und Synergieeffekte mit NADPH-Oxidase-stimulierenden Stoffen (Resveratrol) werden beobachtet. Hier ist ein großes Potenzial für den Einsatz von Synergieeffekten in der Tumortherapie.

### BEISPIELE

### Beispiel 1:

### Wirkung von Superoxiddismutase (SOD) auf die autokrine, durch Reaktive Sauerstoffspezies (ROS) vermittelte Apoptoseinduktion in transformierten Zellen

12 500 Zellen der transformierten Rattenfibroblastenlinie 208Fsrc3 wurden pro 100 µl komplettem Medium in 96-Lochplatten eingesät. Nach dem Anwachsen erfolgte die Zugabe von 20 ng/ml TGF-beta-1 zu allen Ansätzen. Zu den Ansätzen wurden die angegebenen Konzentrationen von Cu-SOD (aus Erythrozyten des Rindes) oder Mn-SOD (aus E.coli) zugegeben. Einige der Ansätze erhielten 50 mM des HOCI-Fängers Taurin (TAU), 150 µM des Peroxidasehemmstoffes 4-Aminobenzoylhydrazid (ABH) oder 10 mM des Hydroxylradikalfängers Mannitol. Nach 22 Stunden wurden die Prozentsätze apoptotischer Zellen aufgrund der klassischen Apoptosemerkmale Kernkondensation, Kernfragmentation oder Membranblebbing jeweils in Doppelansätzen bestimmt.

Figur 1 belegt, dass 208Fsrc3-Zellen in Gegenwart von TGF-beta nach 22 Stunden autokrine Apoptose zeigen. Diese wird durch den HOCI-Signalweg bewirkt, da sie durch den HOCI-Fänger Taurin, den Peroxidasehemmstoff ABH und den Hydroxylradikalfänger Mannitol vollständig gehemmt wird. Steigende Konzentrationen der Cu-SOD und der Mn-SOD im Konzentrationsbereich unter 5 U/ml führen zu einer vollständigen Hemmung der Apoptose, was auf die zentrale Rolle extrazellulärer Superoxidanionen hinweist. Höhere Konzentrationen der Cu-SOD, nicht aber der Mn-SOD führen zu einem erneuten Anstieg der Apoptose in Abhängigkeit von der Konzentration der Cu-SOD. Diese destruktive Wirkung der Cu-SOD hängt ebenfalls von HOCI, Peroxidase und Hydroxylradikalen ab. Sie ist durch die Reaktion des Cu⁺-SOD-Intermediates erklärbar. Dieses entsteht wenn die Cu⁺⁺-Ausgangsform der SOD mit nur einem Superoxidanion reagiert und von diesem reduziert wurde, wobei das Superoxidanion zu molekularem Sauerstoff wurde. Bei Vorliegen hoher SOD-Konzentrationen in Relation zur verfügbaren Superoxidanionenkonzentration scheint der zweite Reaktionsschritt (Cu⁺-SOD + 2 H⁺ + O₂⁻ergibt Cu⁺⁺-SOD + H₂O₂) nicht mehr optimal abzulaufen. Stattdessen wird die Reaktion von Cu⁺-SOD mit HOCl favorisiert, wobei in einer Fenton-ähnlichen Reaktion ein Elektron von der Cu⁺-SOD auf HOCl übertragen wird, wobei dann apoptoseauslösende Hydroxylradikale, Chlorid und die native Cu⁺⁺-SOD entstehen. Das Ergebnis ist eine Glockenkurve der Cu-SOD-Wirkung, mit einem klar definierten Scheitelpunkt der maximalen Hemmwirkung auf die Apoptose. In späteren Abbildungen wird gezeigt, dass die SOD-Konzentration, bei der der Scheitelpunkt erreicht wird von der Konzentration an Superoxidanionen abhängt und daher für eine relative Bestimmung der Superoxidanionenkonzentration ausgezeichnet geeignet ist.

Mn-SOD zeigt diese für die Cu-SOD charakteristische Eigenschaft nicht, nach Erreichen der maximalen Hemmung bleibt diese auch bei einem weiteren Konzentrationsanstieg erhalten. Dies liegt in der Natur des Mn-Ions, das auch als freies Ion, im Gegensatz zum Kupferion nicht für die Fenton-Reaktion geeignet ist.

### Beispiel 2:

### Wirkung von Cu-SOD auf die durch zugegebene Myeloperoxidase (MPO) (A) oder durch den NO-Donor DEA NONOate (B) vermittelte Apoptoseinduktion

Statt der in Beispiel 1 gezeigten autokrinen Apoptoseinduktion wird in dem in Beispiel 2 gezeigten Experiment der HOCI-Signalweg durch Zugabe exogener MPO beschleunigt (Figur 2A) oder der NO/Peroxynitritweg durch Zugabe des schnell zerfallenden NO-Donors DEA NONOate induziert.

Es wurden 12 500 transformierte 208Fsrc3-Zellen pro Ansatz (96-Lochplatte, 100 µl Medium) eingesät. Diese erhielten unter A zusätzlich 200 mU/ml MPO, unter B zusätzlich 1.5 mM DEA NONOate. Kontrollansätze blieben frei von MPO oder DEA NONOate. Im Teil A wurden zusätzlich 100 U/ml Katalase (KAT), 50 mM Taurin (TAU), 10 mM Mannitol (MANN) eingesetzt.

In Figur 2B erfolgte Zugabe von 25 µM des katalytisch wirkenden Peroxynitritzerstörers FeTPPS. Die Doppelansätze unter A wurden nach 5 Stunden, die unter B bereits nach 3 Stunden ausgewertet. Es zeigt sich, dass die Zugabe von MPO den HOCl-Weg beschleunigt. Die Inhibitoren bestätigen die Beteiligung von Wasserstoffperoxid (Hemmung durch Katalase), HOCI (Hemmung durch Taurin), und Hydroxylradikalen (Hemmung durch Mannitol). Es wird wiederum eine Glockenkurve erreicht, deren rechter Teil von der Verfügbarkeit von HOCI und der Wirkung von Hydroxylradikalen abhängt.

Im Versuchsteil B wird durch die Zugabe des NO-Donors Peroxynitrit-abhängige Apoptose induziert. Dazu musste NO mit den Superoxidanionen, die von transformierten Zellen extrazellulär generiert werden reagieren. Diese Reaktion ist naturgemäß durch SOD hemmbar. Der rechte Teil der Glockenkurve, also die destruktive Wirkung hoher Konzentrationen von Cu-SOD ist dadurch erklärbar, dass die Cu⁺-Zwischenform der SOD mit NO zum Nitroxylanion (NO⁻ ) reagiert. Dieses reagiert mit Luftsauerstoff zum Apoptoseinduktor Peroxynitrit. Niedrige Konzentrationen Cu-SOD verhindern also Peroxynitritbildung aus NO, indem sie die dafür benötigten Superoxidanionen aus dem System entfernen, während höhere SOD-Konzentrationen die Bildung von Peroxynitrit fördern, indem sie Nitroxylanionen generieren, die unabhängig von Superoxidanionen direkt mit Luftsauerstoff Peroxynitrit bilden können.

### Beispiel 3:

### Glockenkurve bei der Wirkung von Cu-SOD bei der EUK-8-vermittelten Apoptoseinduktion

Das Katalasemimetikum EUK-8 (Manganese N,N'-bis(salicylidiene)ethylenediamine chloride), ein Salen-Mangan-Komplex besitzt auch Peroxidaseaktivität. Es wurde herausgefunden, dass relativ hohe Konzentrationen von EUK-8 in der Lage sind, HOCI zu synthetisieren. Dabei ist die Affinität des EUK-8 für Wasserstoffperoxid höher als die natürlicher Peroxidase. Damit eignet sich die durch EUK-8 vermittelte HOCl-Synthese zur Apoptoseinduktion in Superoxidanionen-produzierenden Zellen auch bei limitierter Wasserstoffperoxid-Verfügbarkeit (z. B. in Gegenwart von Tumorzell-Katalase).

12 500 208Fsrc3-Zellen in 100 µl (96-Lochplatte) wurden mit steigenden Konzentrationen von Cu-SOD in der Gegenwart von 120 µM EUK-8 versetzt. Nach 5 Stunden wurden die Prozentsätze apoptotischer Zellen in Doppelansätzen bestimmt. Ansätze ohne EUK-8 (aber mit steigenden SOD-Konzentrationen) zeigten zu diesem Zeitpunkt lediglich eine Hintergrundaktivität von weniger als 5 Prozent apoptotischer Zellen (Daten nicht in der Abbildung gezeigt).

Das Ergebnis zeigt, dass die Zugabe von Cu-SOD auch bei der EUK-8-vermittelten Apoptose zu einer Glockenkurve führt.

### Beispiel 4:

### Die durch Cu-SOD hervorgerufene Glockenkurve eignet sich zur relativen Quantifizierung der Superoxidanionenkonzentration

Die angegebenen Zellzahlen (208Fsrc3) in 100 µl Medium wurden mit steigenden Konzentrationen Cu-SOD in der Anwesenheit von 120 µM EUK-8 versetzt. Nach 1.5 Stunden wurden in Doppelansätzen die Prozentsätze apoptotischer Zellen bestimmt. Es zeigt sich eine direkte Abhängigkeit der SOD-Konzentration im Scheitelpunkt von der Zahl der Zellen pro Ansatz. Da diese wiederum die Gesamtkonzentration an verfügbaren Superoxidanionen bestimmt, ist die Korrelation zwischen SOD-Konzentration im Scheitelpunkt und erreichter Superoxidanionenkonzentration gegeben. Die Ergebnisse des Versuches sind in Figur 4 dargestellt.

Aus Gründen der Übersichtlichkeit wurde die Kurve für 6 250 Zellen nicht gezeigt. Deren Scheitelpunkt war bei 0.57 U/ml SOD.

### Beispiel 5:

### Kalibration SOD / Superoxidanionenkonzentration

Die aus dem in Beispiel 4 gezeigten Experiment erhaltenen Daten wurden so aufgetragen, dass die Zellzahl (und damit die relative Superoxidanionenkonzentration) mit der für die maximale Hemmung (Scheitelpunkt) notwendigen SOD-Konzentration korreliert wurde. Es ergibt sich eine streng lineare Wechselbeziehung. Dies zeigt, dass dieses System zur relativen Quantifizierung extrazellulärer Superoxidanionen geeignet ist. Dies ist in Figur 5 dargestellt.

### Beispiel 6:

### Verringerung der Superoxidanionenkonzentration nach gradueller Hemmung der NADPH-Oxidase durch AEBSF

12 500 208Fsrc3-Zellen (100 µl) erhielten die angegebenen Konzentrationen Cu-SOD und 120 µM EUK-8. Zusätzlich wurden die angegebenen Konzentrationen des NADPH-Oxidasehemmers AEBSF (4-(2-Aminoethyl)-benzenesulfonyl fluoride) zugegeben. Kontrollansätze blieben frei von AEBSF. Nach 5 Stunden wurden in Doppelansätzen die Prozentsätze apoptotischer Zellen bestimmt.

Das Ergebnis, dargestellt in Figur 6, zeigt, dass die Hemmung der NADPH-Oxidase durch AEBSF zu einer Verringerung der Superoxidanionenkonzentration führt, da sich die Glockenkurven der Hemmung durch SOD mit ihrem Scheitelpunkt konzentrationsabhängig nach links verschieben. Dabei führt eine Verdoppelung der Inhibitorkonzentration jeweils zu einer Vervierfachung des Rückgangs der Superoxidanionenkonzentration. Konzentrationen von AEBSF die über dem hier gezeigten Messbereich lagen konnten nicht analysiert werden, da sie zu einem Zusammenbruch der Gesamtreaktion führten.

Wichtig ist auch, dass die Wirkung extrazellulärer SOD und die Konsequenz der AEBSF-Wirkung tatsächlich die membranständige NADPH-Oxidase, die extrazelluläre Superoxidanionen generiert, als Zielstruktur definieren.

### Beispiel 7:

### Wirkung von Epothilon B, Malvidinchlorid und Artemisinin auf die extrazelluläre Superoxidanionenproduktion von MKN-45-Zellen

12 500 MKN-45-Tumorzellen in 100 µl Medium wurden mit steigenden Konzentrationen Cu-SOD in Gegenwart von 150 mM 3-AT versetzt. Die Ansätze erhielten zusätzlich, wie angezeigt, Epothilon B, Malvidinchlorid oder Artemisinin. Nach 8 Stunden wurden die Prozentsätze apoptotischer Zellen bestimmt.

Die Figur 7 zeigt, dass nur die höchste Konzentration Epopthilon B zu einer messbaren (vierfachen) Erhöhung der Superoxidanionenproduktion führt, während die niedrigere Epothilonkonzentration, sowie Malvidinchlorid und Artemisinin keine Wirkung auf die Superoxidanionenproduktion zeigten.

### Beispiel 8:

### Steigerung der Superoxidanionenproduktion von MKN-45-Zellen durch Resveratrol

12 500 MKN-45-Zellen pro 100 µl wurden in Gegenwart von 120 µM EUK-8 mit den angegebenen Konzentrationen Cu-SOD versetzt. Kontrollansätze blieben frei von Resveratrol. Zu weiteren Ansätzen wurden die 4 bzw. 20 µg/ml Resveratrol gegeben. Nach 4 Stunden erfolgte die Auswertung.

Das Ergebnis (Figur 8) zeigt, dass Resveratrol im gewählten Konzentrationsbereich eine 8-16-fache Steigerung der Superoxidanionenproduktion bewirkte.

### Beispiel 9:

### Steigerung der Superoxidanionenproduktion von MKN-45-Zellen durch Aktivierung des FAS-Rezeptors mittels Antikörper oder Singulettsauerstoff

A: 12 500 MKN-45-Zellen / 100 µl wurden mit 10 µg/ml eines FAS-Rezeptoraktivierenden monoklonalen Antikörpers gegen FAS-Rezeptor, in Gegenwart oder Abwesenheit von 25 µM Caspase-8-Inhibitors behandelt. Kontrollansätze erhielten keinen Anti-FAS-Antikörper und wurden in Ansätze mit und ohne Caspase-8-Inhibitor unterteilt.
B: 12 500 MKN-45-Zellen / 100 µl wurden mit 1 µg/ml Photofrin, in Gegenwart und Abwesenheit von 25 µM Caspase-8-Inhibitor versetzt. Die Zugaben erfolgten im Halbdunkel. Danach wurden die Ansätze für 20 Minuten mit dem Licht der Neonleuchte der sterilen Werkbank beleuchtet. Dies führt zur Erzeugung von Singulettsauerstoff durch den Photosensitizer Photofrin. Anschließend erfolgte die Zugabe von 100 mM 3-AT und der angegebenen Konzentrationen CuSOD. Nach 5 Stunden wurden in den Doppelansätzen die Prozentsätze apoptotischer Zellen bestimmt.

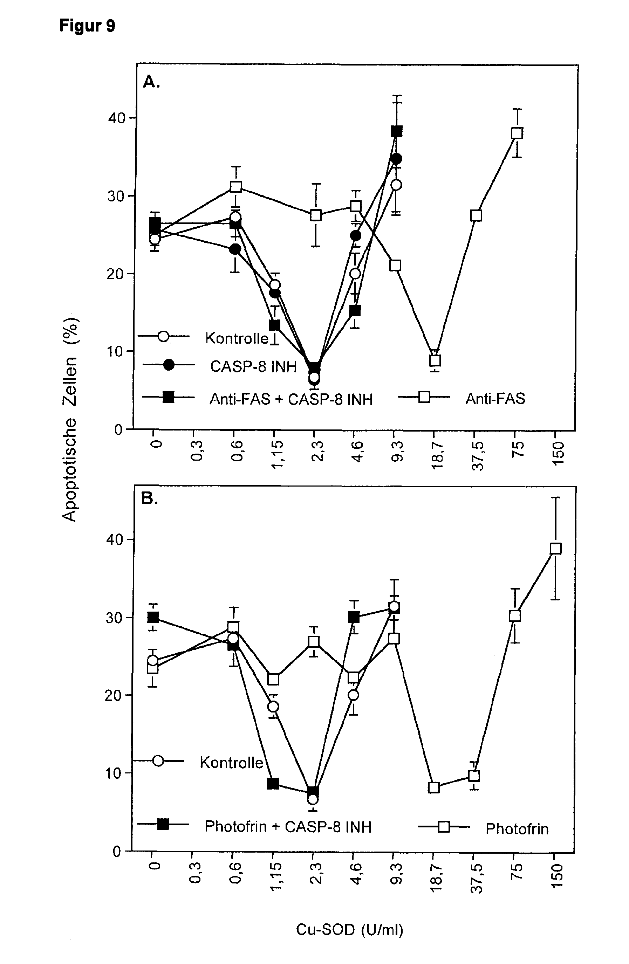

Die Figur 9 zeigt, dass die Aktivierung des FAS-Rezeptors mittels monoklonalem Antikörper zu einer sehr deutlichen Steigerung der Superoxidanionenproduktion führt. Die Spezifität dieser Wirkung wird durch die Hemmung mittels Caspase-8-Inhibitor belegt. Caspase-8 wird durch den FAS-Rezeptor aktiviert. Es ist wichtig, zu wissen, dass in MKN-45-Zellen die Aktivierung des FAS-Rezeptors nicht ausreicht, um Apoptose zu induzieren, da die Rezeptordichte zu gering ist.

Die Figur 8 zeigt weiterhin, dass Singulettsauerstoff eine analoge Wirkung zeigt wie der monoklonale Antikörper gegen FAS-Rezeptor. Die Aufhebung der Wirkung des Singulettsauerstoffs mittels Caspase-8-Inhibitor belegt, dass diese durch den FAS-Rezeptor vermittel wurde.

### Beispiel 10:

### Hemmung der NO-Dioxygenase (NOD) durch Epothilon B

12 500 MKN-45-Zellen in 100 µl Medium wurden mit 200 mM 3-AT, 2.4 mM NAME, 25 µM EUK-134 und den angegebenen Konzentrationen Epothilon B ("EPO") versetzt. Kontrollansätze erhielten keine Epothilon. Anschließend erfolgte die Zugabe der angegebenen Konzentrationen des NO-Donors DEANONOate und die Ansätze wurden für weitere 2 Stunden bei 37 °C inkubiert bevor die Prozentsätze apoptotischer Zellen bestimmt wurden.

Die Figur 10 zeigt, dass alle hier eingesetzten Konzentrationen Epothilon B zu einer Steigerung der DEA-NONOATE-abhängigen Apoptose führten. Dies ist durch die Hemmung des Verbrauchs von NO durch die NOD erklärbar. Daraus resultiert eine erhöhte Verfügbarkeit von NO im System.

### Beispiel 11:

### Hemmung der NOD durch Taxol und Diallydisulfid (DADS)

Das Experiment wurde so durchgeführt, wie in Abbildung 11 beschrieben, mit dem Unterschied, dass die angegebenen Konzentrationen DADS beziehungsweise Taxol eingesetzt wurden und die Bestimmung der apoptotischen Zellen nach 3 Stunden erfolgte.

Die Figur 11 zeigt, dass auch DADS und Taxol zu einer Erhöhung der verfügbaren NO-Konzentration führten. Dies ist durch Hemmung der NOD erklärbar.

### Beispiel 12:

### Synergistische Wirkung zwischen Resveratrol und Arginin bei der Sensitivierung von Tumorzellen für Apoptoseinduktion

12 500 MKN-45-Zellen in 100 µl Medium wurden mit den angegebenen Konzentrationen Arginin in Kombination mit 0.2 bzw. 20 µM Resveratrol angesetzt. Kontrollansätze erhielten Arginin zwischen 0 und 5 mM, blieben aber frei von Resveratrol. Nach 4.5 Stunden wurden die Prozentsätze apoptotischer Zellen bestimmt (Doppelansätze).

Die Figur 12 zeigt, dass Arginin (das Substrat der NO-Synthase) zu einer konzentrationsabhängigen Apoptoseinduktion in den Tumorzellen führt. [Parallel durchgeführte Kontrolluntersuchungen (Daten hier nicht gezeigt) belegen, dass dies durch Wiederherstellung des interzellulären ROS-Signaling nach Zerstörung der protektiven, membranständigen Katalase der Tumorzellen bewirkt wird. Bei der Zerstörung spielt aus Peroxynitrit und Wasserstoffperoxid generierter Singulettsauerstoff eine zentrale und sehr frühe Rolle].

Resveratrol, das im gewählten Konzentrationsbereich und nach 4.5 Stunden kaum mehr als Backgroundapoptose induziert, führt zusammen mit niedrigen Argininkonzentrationen zu einem sehr beeindruckenden synergistischen Effekt. Dieser beruht auf dem Zusammenspiel der Stimulation der NADPH-Oxidase durch Resveratrol und der Erhöhung der NO-Synthese durch Arginin.

### Beispiel 13:

### Rolle von FAS für die Apoptoseinduktion durch Arginin in Ab- oder Anwesenheit von Resveratrol

Das Experiment wurde wie in Beispiel 12 beschrieben durchgeführt. Zusätzlich wurde zu den angegebenen Kombinationen von Arginin und Resveratrol 25 µM Caspase-8-Inhibitor zugegeben oder nicht. Auswertung nach 4.5 Stunden.

Die Figur 13 zeigt, dass die Apoptose-auslösende Wirkung durch Arginin allein strikt von der Beteiligung eines Caspase-8-vermittelten Schrittes abhängt. Bei 0.2 µg/ml Resveratrol wird die Wirkung hoher Konzentrationen Arginin vom FAS-Rezeptor und seiner nachgeschalteten Caspase-8 unabhängig, während bei den kleineren Argininkonzentrationen diese Abhängigkeit weiterhin bestehen bleibt. Schließlich zeigt sich bei 20 µg/ml Resveratrol in Kombination mit allen Argininkonzentrationen eine weitgehende Unabhängigkeit von FAS und Caspase-8.

### Beispiel 14:

### Direkter Nachweis der durch Taxol unter Beteiligung von Singulettsauerstoff bewirkten Katalaseinaktivierung von Tumorzellen

A: Darstellung der protektiven Wirkung der Katalase in Figur 14A
   25 000 Zellen der humanen Lymphomlinie Gumbus / 100 µl Medium wurden ohne 3-AT oder in Gegenwart von 50 mM bzw. 100 mM des Katalaseinhibitors 3-AT mit den angegebenen Konzentrationen Wasserstoffperoxid versetzt. Nach 1.5 Stunden wurde in Doppelansätze die Apoptoseinduktion bestimmt.
B: Gumbuszellen wurden ohne Taxol (Kontrolle) oder mit 10 µg/ml Taxol für 30 Minuten bei 37 °C vorinkubiert. Parallelansätze waren entweder frei von weiteren Stoffen oder enthielten 2 mM Histidin (Singulettsauerstoff-Fänger), 25 µM FeTPPS (katalytisch wirkender Peroxynitritzerstörer) oder 25 U/ml Katalase (CAT). Nach der Vorinkubation wurden die Zellen abzentrifugiert, in frischem Medium aufgenommen und mit den angegebenen Konzentrationen Wasserstoffperoxid versetzt. Nach 1.5 Stunden erfolgte die Auswertung in den Doppelansätzen.

Die Figur 14 zeigt, dass Gumbus einen deutlichen Schutz vor exogenem Wasserstoffperoxid aufweisen, der auf ihrer Katalase beruht, da er durch den Katalaseinhibitor 3-AT aufgehoben werden kann.

Die Vorbehandlung mit Taxol hat den gleichen Effekt wie 3-AT. Die Hemmwirkung hält auch nach Wegwaschen des Taxols an und ist daher am besten als irreversible Inaktivierung erklärbar. Dabei spielt Singulettsauerstoff eine zentrale Rolle. Die Interaktion von Wasserstoffperoxid und Peroxynitrit stellt die wahrscheinlichste Quelle für den Singulettsauerstoff dar, wie die Hemmdaten zeigen.

### Beispiel 15:

### Synergistische Wirkung zwischen Taxol und Resveratrol

12 500 MKN-45 Zellen in 100 µl wurden mit 10 µg/ml Taxol oder 0.013 µg/ml Taxol mit oder ohne Kombination mit den angegebenen Konzentrationen Resveratrol versetzt. Weitere Ansätze erhielten keine Zusätze (Kontrolle) oder die verschiedenen Resveratrolkonzentrationen allein. Nach 4.5 Stunden wurden in Doppelansätzen die Prozentsätze apoptotischer Zellen ermittelt.

Die Figur 15 zeigt, dass 0.013 µg/ml Taxol oder jede der angegebenen Konzentrationen Resveratrol für sich allein keine Apoptose induzierten. 10 µg/ml Taxol zeigten deutliche Apoptoseinduktion. Die Kombination zwischen 0.013 µg/ml Taxol mit Resveratrol führt zu einem beachtlichen synergistischen Effekt.

### Beispiel 16:

### Synergistische Wirkung zwischen Epothilon B und Resveratrol

12 500 MKN-45-Zellen in 100 µl Medium erhielten entweder keine Zugabe von Substanzen, 25 ng/ml Epothilon B, 0.75 ng/ml Epothilon B, 25 µg/ml Resveratrol oder die Kombination aus 0.75 ng/ml Epothilon B mit 25 µg/ml Resveratrol. Nach 3 Stunden wurden in Doppelansätzen die Prozentsätze apoptotischer Zellen bestimmt.

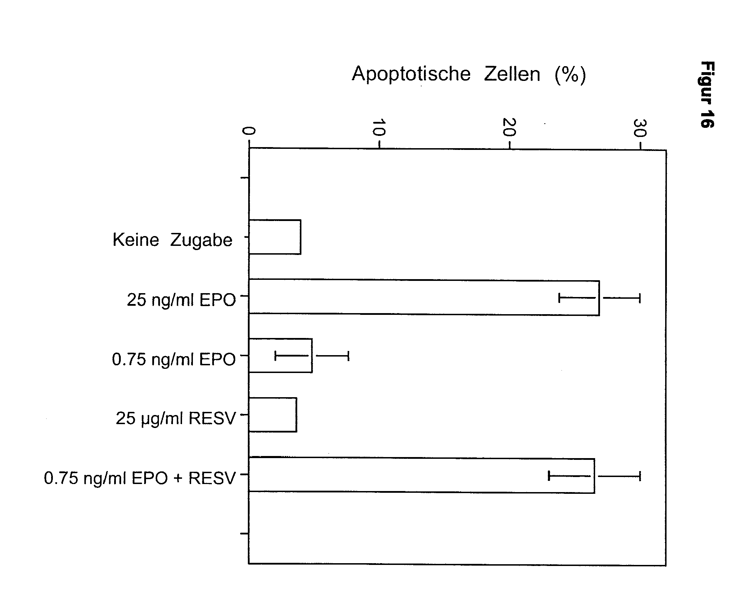

Die Figur 16 zeigt, dass weder die niedrige Epothilon B-Konzentration noch Resveratrol für sich alleine Apoptose induzieren konnten, während sie in Kombination einen synergistischen Effekt bewirkten, der eine Apoptoseinduktion erreichte, die mit der der hohen Epothilonkonzentration vergleichbar ist.

### Beispiel 17:

### Synergistische Wirkung zwischen Cyanidinchlorid und Resveratrol

12 500 MKN 45-Zellen in 100 µl Medium erhielten keine Zugabe oder die angegebenen Zusätze. Nach 3 Stunden erfolgte die Auswertung der Doppelansätze.

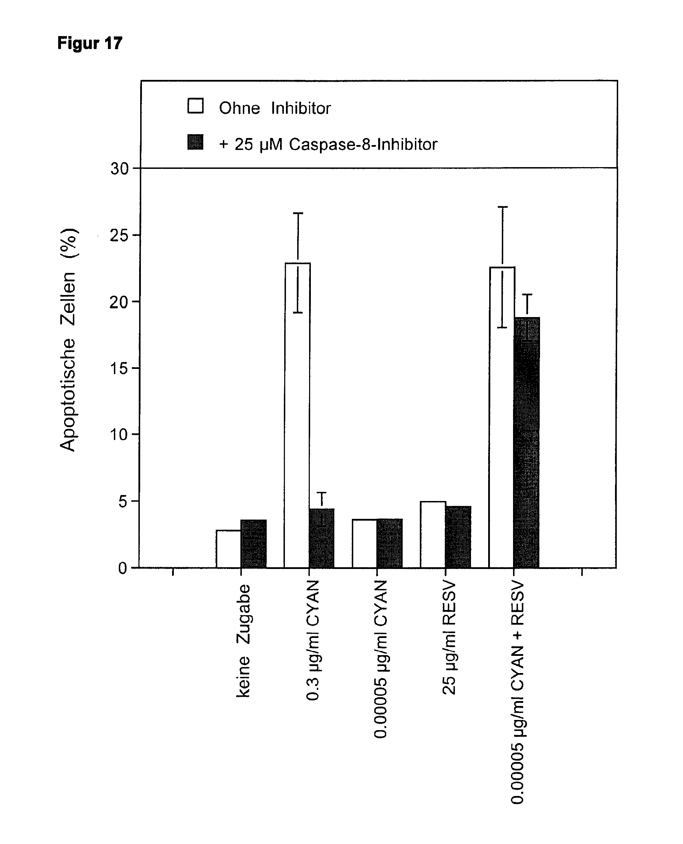

Die Figur 17 zeigt einen bemerkenswerten synergistischen Effekt zwischen Cyanidinchlorid und Resveratrol. Die Wirkung der hohen Cyanidinchloridkonzentration ist von Caspase-8 abhängig, während dies für den synergistischen Effekt kaum zutrifft.

### Beispiel 18:

### Hemmung des interzellulären ROS-Signalings durch Katalase

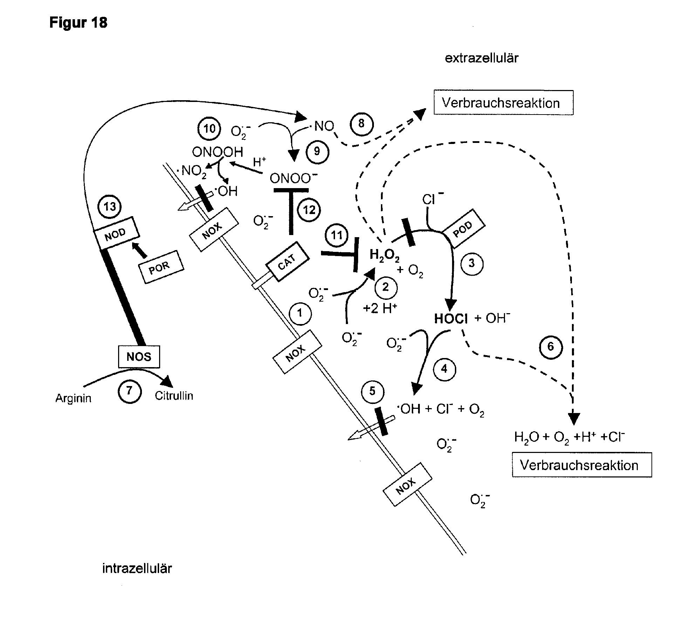

Die Figur 18 zeigt links den intra-, rechts den extrazellulären Raum einer Tumorzelle. In der Zellmembran befindet sich die aktivierte NADPH-Oxidase NOX-1 (1), die extrazellulär Superoxid-anionen generiert. Diese dismutieren spontan zu Wasserstoffperoxid und Sauerstoff (2). In transformierten Zellen ohne membranständige Katalase (hier nicht gezeigt) wird Wasserstoffperoxid wird einer freien Peroxidase (POD) in HOCI umgesetzt (3), welches mit Superoxidanionen zu Apoptose-induzierenden Hydroxylradikalen reagiert (4,5). Bei relativem Überschuss an Wasserstoffperoxid kommt es zur Verbrauchsreaktion von HOCI (6). NO-Synthase (NOS) generiert NO (7), welches entweder von Wasserstoffperoxid verbraucht wird (8) oder mit Superoxidanionen zu Peroxynitrit reagiert (9). Nach Bildung von Peroxynitritsäure und deren Zerfall in Hydroxylradikale und NO₂ kommt es zur Apoptoseinduktion (10). Tumorzellen besitzen ausreichend membranständige Katalase, um durch das Zerstören von Wasserstoffperoxid (11) oder Peroxynitrit (12) das interzelluläre ROS-Signaling vollständig zu unterbinden. Die beiden untergeordneten Signalwege Nitrylchloridweg und Metall-katalysierte Haber-Weiss-Reaktion sind in dem Schema nicht berücksichtigt, sie werden jedoch wegen ihrer Abhängigkeit von Wasserstoffperoxid durch Katalase ebenfalls vollständig gehemmt.

Eine Schlüsselrolle nimmt die NO-Dioxygenase (NOD) (13) ein. Sie wandelt einen erheblichen Teil des durch NOS synthetisierten NO in Nitrat um und wird selbst durch Cytochrom P450 Oxidoreduktase (POR) moduliert.

### Beispiel 19:

### Sensitivierung von Tumorzellen für interzelluläres ROS-Signaling

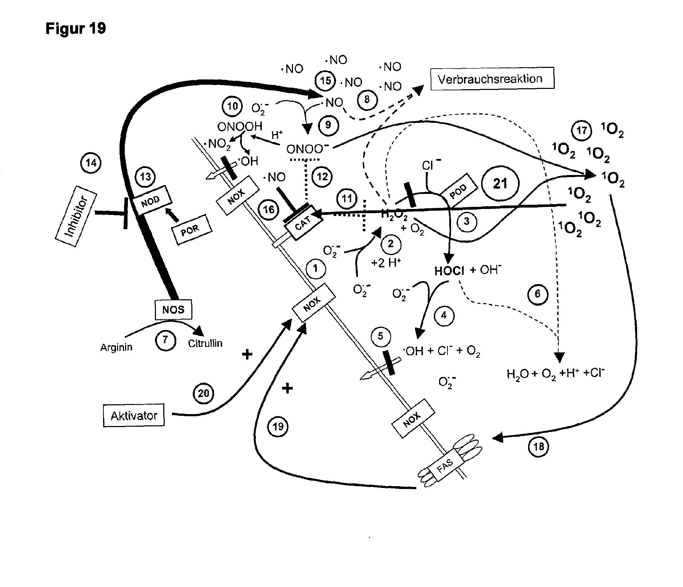

Der Zusammenhang zwischen den komplexen Reaktionen wird in Figur 19 dargestellt. Die (potenziellen) interzellulärem Signalwege 1-13 entsprechen denen, die in Figur 18 beschrieben wurden.

Trifft ein Inhibitor der NOD auf eine Tumorzelle, kommt es zu einem sprunghaften Anstieg der verfügbaren NO-Konzentration (14, 15). Daraus resultiert möglicherweise eine transiente und partielle Hemmung der Katalase (16), in jedem Fall aber ein Anstieg der Peroxynitritkonzentration. Als Konsequenz kommt es zur Reaktion von Peroxynitrit mit Wasserstoffperoxid (17), wobei Singulettsauerstoff gebildet wird. Falls dieser in ausreichender Konzentration gebildet wird, kann sofort die Inaktivierung von Katalase erfolgen (21), wodurch nachfolgend Apoptoseinduktion durch interzelluläres ROS-Signaling ermöglicht wird. Ist die Singulettsauerstoffkonzentration zu gering für die direkte Inaktivierung von Katalase, erfolgt zunächst Aktivierung des FAS-Rezeptors durch Singulettsauerstoff (18). Dies führt zu einer Aktivierung der NADPH-Oxidase NOX-1 (19). Als Konsequenz wird die Konzentration an Wasserstoffperoxid und nachfolgend die des Singulettsauerstoffs erhöht und Katalase nun nach diesem Amplifikationsschritt inaktiviert. Aktivatoren von NOX-1, wie zum Beispiel Resveratrol führen zur gleichen Amplifikationswirkung wie die Aktivierung des FAS-Rezeptors (20). Die gleiche Wirkung wie durch Hemmung der NOD (14) kann durch Erhöhung des Argininspiegels durch Zugabe der Aminosäure oder Hemmung der Arginase oder durch Induktion der Expression von NOS erreicht werden (nicht im Schema gezeigt).

Die parallele Erhöhung der verfügbaren NO-Konzentration und der Superoxidanionkonzentration könnte einen neuen Ansatz zur wirkungsvollen Sensitivierung und ROS-gesteuerter Selbstzerstörung von Tumorzellen eröffnen, bei dem aufgrund des Synergieeffektes die Wirkstoffe im nebenwirkungsfreien Konzentrationsbereich eingesetzt werden können. Die Kenntnisse der Signalwege und die Verfügbarkeit entsprechender Testsysteme sollte auch die Synthese von Hybridmolekülen ermöglichen, die beide benötigten Aktivitäten in einem Molekül vereinigen.

## Patentansprüche

1. Pharmazeutische Zusammensetzung, **dadurch gekennzeichnet, dass** sie eine pharmazeutisch wirksame Menge an wenigstens einem Wirkstoff enthält, der die verfügbare NO-Konzentration in der Zelle erhöht, zusammen mit wenigstens einem Wirkstoff, der die NADPH-Oxidase stimuliert.

2. Pharmazeutische Zusammensetzung nach Anspruch 1, **dadurch gekennzeichnet, dass** der Wirkstoff, der die NADPH-Oxidase stimuliert, ausgewählt ist aus Resveratrol, Transforming growth factor ß und/oder Angiotensin II.

3. Pharmazeutische Zusammensetzung nach Anspruch 1 oder 2, **dadurch gekennzeichnet, dass** der Wirkstoff, der die verfügbare NO-Konzentration in der Zelle erhöht, keine gleichzeitige Wirkung auf die NADPH-Oxidase ausübt.

4. Pharmazeutische Zusammensetzung nach einem der Ansprüche 1-3, **dadurch gekennzeichnet, dass** der Wirkstoff, der die NO-Konzentration in der Zelle steigert, ausgewählt ist aus Arginin und oder Arginasehemmstoffen, insbesondere NOHA und oder NOR-NOHA.

5. Pharmazeutische Zusammensetzung nach einem der Ansprüche 1-3, **dadurch gekennzeichnet, dass** der Wirkstoff, der die verfügbare NO-Konzentration in der Zelle erhöht, die NO-Synthase induziert.

6. Pharmazeutische Zusammensetzung nach Anspruch 5, **dadurch gekennzeichnet, dass** der Wirkstoff, der die NO-Synthase induziert, Interferon Y ist.

7. Pharmazeutische Zusammensetzung nach einem der Ansprüche 1-3, **dadurch gekennzeichnet, dass** der Wirkstoff, der die verfügbare NO-Konzentration in der Zelle erhöht, eine hemmende Wirkung auf die NO-Dioxigenase hat und ausgewählt ist aus
a) Flavonoiden, insbesondere
Xanthohumol, Isoxanthohumol, 6-Prenylnaringenin,
8-Prenylnaringenin, Quercetin, Quercitrin, Isoquercetin, Rutin, Taxifolin, Hyperosid, und/oder
b) Anthocyanen, insbesondere
Cyandidinchlorid, Malvidinchlorid, Malvidin-3-O-galaktosid, Pelargonin, Peonidinchlorid, Pelargonidin, und/oder
c) Fettsäuren, insbesondere
Palmitinsäure, Stearinsäure, Myristinsäure, und/oder
d) Azolen, insbesondere
Biconazol, Econazol, Fluconazol, Itraconazol, Ketoconazol,
Miconazol; Sulconazol, und/oder
e) Artemisinin, Chloroquin, Primaquin.

8. Pharmazeutische Zusammensetzung nach einem der Ansprüche 1-7 zur Verwendung bei der Behandlung einer Krebserkrankung.

9. Pharmazeutische Zusammensetzung nach Anspruch 8 zur Verwendung bei der Behandlung von Magenkrebs.
